Europäisches Patentamt

European Patent Office

Office européen des brevets

(11) Publication number: **0 292 818**
**A2**

(12) **EUROPEAN PATENT APPLICATION**

(21) Application number: 88107774.7

(51) Int. Cl.4: **A61K 35/52**

(22) Date of filing: 14.05.88

(30) Priority: 28.05.87 IT 2070187

(43) Date of publication of application:
30.11.88 Bulletin 88/48

(84) Designated Contracting States:
AT BE CH DE ES FR GB GR IT LI LU NL SE

(71) Applicant: **CONSIGLIO NAZIONALE DELLE RICERCHE**
**Piazzale Aldo Moro, 7**
**I-00185 Roma(IT)**

Applicant: **OSPEDALE MAGGIORE POLICLINICO**
**Via Francesco Sforza , 28**
**I-20100 Milano(IT)**

(72) Inventor: **The other inventor has agreed to waive his entitlement to designation**

(74) Representative: **Minoja, Fabrizio**
**Studio Consulenza Brevettuale Via Rossini, 8**
**I-20122 Milano(IT)**

(54) **Pharmaceutical compositions with immunomodulating activity.**

(57) Pharmaceutical compositions with immunomodulating activity are described, in particular immunosuppressive activity, containing as active principle a peptide fraction with low molecular weight (about 1000) extracted from seminal plasma.

EP 0 292 818 A2

# PHARMACEUTICAL COMPOSITIONS WITH IMMUNOMODULATING ACTIVITY

The present invention concerns pharmaceutical compositions with immunomodulating activity containing as active principle a peptide fraction with low molecular weight ($\approx$ 1000) extracted from seminal plasma.

This fraction, referred to hereinafter for brevity as "SPI", has already been described in the literature as having inhibitory activity on RNA and DNA synthesis in reconstituted systems (cellfree) and in nuclei isolated from cells of various tissues (Archives of Andrology 1984, 13, 261-267).

This SPI has furthermore been demonstrated therapeutically effective in the treatment of cataract and senescence-related diseases.

It has now, surprisingly, been found that SPI has immunomodulating activity, more specifically immunosuppressive activity, in vitro and in vivo, so that its use in human treatment could be beneficial.

There is an obvious, much felt requirement for new agents able to interact with the immune system thus providing adequate therapeutic means to control rejection of organ and tissue transplants, and to treat viral, autoimmune and some tumoral diseases.

This task is hampered by the fact that the human immunocompetent system is very complex and still not completely understood. It is now accepted that there are two types of immunity:

(i) humoral, mediated by antibodies (B-cell compartment) and

(ii) cellular, mediated by specific cells (T-cell compartment).

Both the antibodies and the specific cells directed against foreign bodies like pathogenic microbial agents or cells in tumoral transformation provide an effective defense of the organism.

However, the complexity of the immune system derives from the existence of an intricate network system to regulate communications which is able to determine multiple and sometimes contrasting effects. This regulatory mechanism may greatly enhance or suppress the immune response according to the needs of the organism by the use of soluble mediators able to enhance or suppress the division or differentiation of immunocompetent cells. These soluble mediators are in their turn produced by regulatory T cells (helper/suppressor).

From this it is evident that enhancement and suppression of the immune response may be achieved with the use of single soluble mediators deriving respectively from the helper or suppressor cells. This explains the enormous interest in isolating such products.

During the course of studies to investigate the biological properties of the SPI factor it was observed that this factor showed an inhibitory activity on human lymphocyte transformation, index of immunosuppressive activity.

Experiments were performed on human mononuclear peripheral blood cells obtained as already described (Journal of Immunology 1979, 122, 1658-1662), diluting 10 ml of heparinized blood with 20 ml of Hanks' balanced saline solution (HBSS) in a plastic test-tube (30 x 115 mm). 15 ml of Ficoll-Urovison gradient was stratified underneath and the test-tube was then centrifuged at 400 g at the interface for 30 minutes. The cells removed from the interface were washed three times with HBSS and suspended at a concentration of 2.5 x 10⁵ cells/ml in RPMI 1640 medium with Hepes (20 mM), penicillin (100 IU/ml), streptomycin (100 mcg/ml) and 10% of AB serum.

Lymphocyte transformation was obtained using highly purified PHA at a final concentration of 0.2 mcg (a suboptimal concentration as determined previously from a dose:response curve) according to Kay J.E. et al. (Cell. Immunol. 1984, 87, 217-224) in a microculture system of 5 x 10⁴ cells in round-bottomed, tissue culture microplates. The cells incubated in a humidified atmosphere at 37°C for 72 hours were labeled for the last 16 hours with ³H-thymidine, cultured, and the radioactivity was measured with a scintillation counter.

The SPI factor, purified as described below, was added to the cultures in varying doses at zero time. As shown in the following table, the SPI factor induced inhibition of lymphocyte transformation in a dose-dependent fashion.

All possible toxic effects of SPI on the cells were studied by measuring cell viability and the absolute number of cells in culture during the experiment. Cell viability was over 90% in all the cultures, whereas a cell loss of under 10% was observed in the samples with SPI but only in the cultures with the highest concentration of SPI (24 mcg/culture).

These findings of immunosuppressive activity in vitro were confirmed in other experimental models in vivo.

TABLE. Inhibition of lymphocyte transformation by seminal factor SPI.

| SPI dose (mcg/culture) | cpm x $10^{-4}$ |
|---|---|
| 0.75 | 3.5 ± 0.2 |
| 1.5 | 3.1 ± 0.1 |
| 6 | 1.1 ± 0.2 |
| 24 | 0.2 ± 0.1 |

Considering the above findings, this product could be used in clinical practice in all pathological conditions in which it is necessary to suppress the immune response, that is:

A. In deficiencies due to suppressor cell activity.

For example, (i) in allergic disorders due to IgE like hay fever, (ii) in some malignant diseases due to proliferation of lymphocyte clones like leukemias and plasmacytomas.

B. In deficiencies of suppressor cell activity associated with excess or inappropriate activity of helper cells.

For example, in some autoimmune diseases like systemic lupus erythematosus, rheumatoid arthritis and autoimmune hemolytic anemias.

C. In organ and tissue transplantation.

In this case administration of the product does not have the same objective of restoring the immune response to normal as in the preceding cases, since it is "normal" for the immune response to attempt to reject the transplant, but it has the aim of inducing tolerance by reducing the damaging effects of the immune response on the transplant. Instead, in the case of hemopoietic bone marrow transplants the product would have the function of reducing graft versus host disease (GVHD).

D. In some infectious diseases with immune aggressivity.

A typical example is chronic active hepatitis. Furthermore, among the infectious diseases, a beneficial effect may be hypothesized in the acquired immunodeficiency syndrome (AIDS/HIV) and similar forms. In fact, it is known that the AIDS viruses infect the helper cells of the host and multiply when these cells are stimulated. Administration of the product, suppressing the proliferation of helper cells, would reduce the number of multiplying viruses, thus breaking the loop: virus infecting helper cells/cell proliferation-viral multiplication/new viruses that infect other cells with consequent progression of the disease.

For the anticipated therapeutic uses, the SPI factor may conveniently be administered to patients by the oral or parenteral route in the form of appropriate pharmaceutical compositions, which can be prepared using the conventional techniques and excipients such as those described in "Remington's Pharmaceutical Sciences Handbook", Hack Pub. Co., New York; USA. Examples of these formulations include capsules, tablets, bottles or ampules containing lyophilized powders to be reconstituted before use with appropriate solvents, possibly controlled-release forms.

The dosages and treatment protocols will depend obviously on the use considered and cn the conditions of the patient; in principle from 1 to 1000 μg/kg/dose by the parenteral route and from 10 to 1000 μg/kg/dose by the oral route will be sufficient although lower or higher dosages cannot be excluded a priori in consideration on the one hand of the possibilities of further purification of the active principle and on the other of its limited toxicity.

Although the SPI factor has already been described, we report below, as an example, a description of its extraction and characterization. This example must obviously not be understood as limiting.

## EXAMPLE

Bovine seminal plasma is obtained by centrifugation of sperm in toto (conserved a -80°C from the time of ejaculation) at 8000 x g for 30 minutes at +2°C. Ultrafiltration by filtering membrane with porosity of under 5 nm (cutoff 10,000 daltons) is then performed at +2°C.

An amount of lyophilized ultrafiltrate equivalent to 100 ml of seminal plasma is extracted in the minimum volume of glacial acetic acid, then centrifuged at 9500 x g for 30 minutes at +2°C. The surnatant is mixed, drop by drop and with vigorous agitation, to 9 volumes of cold, peroxidase-free diethyl ether. The precipitate that forms is washed abundantly in a Bücher funnel with cold ethyl ether, pump dried, and lyophilized. A second acetic extraction and precipitation with diethyl ether is then performed in the same conditions as described above, the precipitate, washed with ether, is vacuum evaporated, solubilized in water, and lyophilized.

The raw material thus obtained, solubilized in a small volume, is separated by gel filtration on Sephadex G-15 in acetic acid 0.1 M (column of ⌀ 2.6 x 110 cm, flow rate 60 ml/hour).

A fraction of acid nature, which elutes from the column between 1.6 and 1.9 times the empty volume of the column, contains the bulk of the activity inhibiting DNA and RNA synthesis.

After lyophilization and neutralization, this fraction is separated by gel filtration on Bio-Gel P-2 equilibrated in acetic acid 0.1 M (column of ⌀ 1.6 x 110 cm, flow rate 22 ml/hour). A fraction, also this a delayed fraction, contains practically all the inhibitory activity.

After lyophilization, aliquots of the active fraction are further purified by high performance liquid chromatography in 4% acetic acid or in trifluoroacetic acid at pH 2, on LiChrosorb RP-18 column, 7 μm, 10 x 250 (h) mm.

A delayed fraction of acid nature is active in the tests reported.

This fraction may be further purified in water, on Sephadex G-10 (column of ⌀ 1.8 x 180 cm, flow rate 40 ml/hour).

The characteristics of the fraction obtained are as follows.
Amino acid residues:

pyroglutamic acid at the N terminal, alanine (2nd amino acid?), glutamic acid or glutamine, aspartic acid or asparagine (at the C terminal?), serine, glycine, threonine
It inhibits DNA polymerization and RNA transcription in vitro.
It binds and stabilizes the DNA double helix.
It is resistant to heat and to acid denaturation.
It is resistant to trypsin.
It is sensitive to pronase and to pyroglutamate aminopeptidase.

## Claims

1. Pharmaceutical compositions with immunomodulating activity containing as active principle the peptide fraction with low molecular weight (about 1000) extracted from seminal plasma.

2. Compositions according to claim 1, appropriate for administration by the oral or parenteral route.

3. Compositions according to claims 1 or 2, appropriate for administration of from 1 to 1000 μg/kg/dose of active principle.

4. Use of the peptide fraction with low molecular weight extracted from seminal plasma for the preparation of a medicament with immunomodulating activity.